# FASCICULE DE BREVET EUROPEEN

(11) **EP 2 780 768 B1**
(45) Date de publication et mention de la délivrance du brevet: **24.02.2021**
(21) Numéro de dépôt: 12797952.4
(22) Date de dépôt: 16.11.2012
(51) Int. Cl.: G05B 19/19, G05B 19/402, G01N 35/10, C12M 1/26, G01N 1/02, C12Q 1/24

(54) **PROCÉDÉ OPTIQUE POUR PILOTER LE DÉPLACEMENT D'UN OUTIL DE PRÉLÈVEMENT**
OPTISCHES VERFAHREN ZUR STEUERUNG DER BEWEGUNG EINES PROBENAHMEWERKZEUGS
OPTICAL METHOD FOR CONTROLLING THE MOVEMENT OF A SAMPLING TOOL

(30) Priorité: 17.11.2011 FR 1160497
(43) Date de publication de la demande: 24.09.2014
(73) Titulaire: Biomérieux, 69280 Marcy l'Étoile (FR)
(72) Inventeur: DECAUX, Dominique, F-69630 Chaponost (FR); MONTVERNAY, Régis, F-69004 Lyon (FR); PINSTON, Frédéric, F-38000 Grenoble (FR)
(74) Mandataire: Schoen, Elsa Imilia
(86) Numéro de dépôt international: PCT/FR2012/052655
(87) Numéro de publication internationale: WO 2013/072643

(56) Documents cités:
- DE-A1-102004 030 881
- US-A1- 2006 043 287
- US-B2- 7 881 533

## Description

L'invention concerne un procédé pour effectuer un prélèvement de matière biologique sur une surface de référence à l'aide d'un outil de prélèvement, le procédé permettant de piloter le déplacement d'un outil de prélèvement vers une surface de référence et/ou d'une surface de référence vers un outil prélèvement, un dispositif pour la mise en œuvre de ce procédé, ainsi qu'un appareil de prélèvement de matière biologique comprenant ledit dispositif.

En d'autres termes, le procédé selon la présente invention permet de piloter le déplacement d'un outil de prélèvement de matière biologique, tel qu'une pipette, en direction d'une surface de référence. Ladite surface de référence peut notamment consister en une boîte de Pétri remplie de gélose sur laquelle est mise en culture la matière biologique d'intérêt.

Par « matière biologique », l'on entend toute matière contenant des informations génétiques et qui est autoreproductible dans un système biologique. A ce titre, l'on peut notamment citer toutes cultures cellulaires, bactériennes ou virales.

Nombre de solutions techniques existent afin de prélever de la matière biologique, telle qu'une colonie bactérienne mise en culture sur une boîte de Pétri, et ce en vue de procéder à des analyses ultérieures.

Ce prélèvement de matière biologique peut notamment être effectué grâce à des outils de prélèvement « passifs », par exemple un outil pourvu en son extrémité d'un élément en coton destiné à entrer en contact avec une colonie bactérienne. De façon alternative, des outils de prélèvement « actifs » sont utilisés, comme par exemple des pipettes utilisant le principe d'aspiration/refoulement. Ces outils de prélèvements peuvent être manuels ou automatisés.

Afin de ne pas fausser l'analyse de la matière biologique prélevée, il est important de ne prélever que la matière biologique d'intérêt en s'abstenant de prélever toute substance étrangère à cette dernière, telle que des substances provenant du milieu de culture *per se.* Cette substance étrangère peut être qualifiée de substance contaminante. En matière de prélèvement bactérien sur une boîte de Pétri, il convient donc d'éviter de prélever de la gélose en même temps que la matière biologique d'intérêt.

En effet, la gélose non intentionnellement prélevée peut perturber aussi bien l'analyse quantitative que qualitative de la matière biologique prélevée.

En matière d'analyse quantitative, les résidus issus de milieu gélosé peuvent fausser la mesure de la densité optique d'un échantillon comprenant la suspension bactérienne en opacifiant artificiellement cette suspension bactérienne.

Concernant l'analyse qualitative, par exemple l'analyse par spectrométrie de masse, les résidus de gélose vont induire la détection de composés étrangers à la matière biologique d'intérêt qui génère un bruit de fond perturbant l'analyse.

Par ailleurs, il est possible qu'une colonie bactérienne contaminante prolifère sous une colonie bactérienne d'intérêt. Dans ce cas de figure, il est important, lors du prélèvement de la colonie bactérienne d'intérêt, de ne pas prélever de bactéries contaminantes afin de ne pas fausser l'analyse correspondante.

Plusieurs dispositifs ont été développés dans l'art antérieur, afin de tenter d'éviter de tels phénomènes de contamination essentiellement dus à un enfoncement trop important de l'outil de prélèvement dans le milieu de culture, solide, liquide ou semi-solide, par exemple un enfoncement de l'embout d'une pipette dans de la gélose.

Un premier type de dispositif de l'art antérieur permet la détection du contact de l'outil de prélèvement avec une surface en utilisant des propriétés capacitives, par contact électrique.

Premièrement, c'est le cas de l'aiguille métallique non jetable, qui remplit la fonction d'électrode, la deuxième électrode étant le liquide dans lequel l'aiguille va plonger. Lorsque cette aiguille rentre en contact avec l'échantillon biologique une variation d'impédance, capacitance ou résistance, après amplification, déclenche l'arrêt du moteur de descente d'aiguille. Les dispositifs et procédés utilisant cette technique sont notamment décrits dans le document US 4,818,491.

Deuxièmement, on peut utiliser un cône ou embout conducteur qui est enfichable et donc jetable après chaque prélèvement par la pipette associée au cône. Ce dernier est fabriqué dans un matériau plastique chargé en particules de carbone, la liaison électrique est assurée par le préhenseur de cônes qui lui sera obligatoirement conducteur.

Troisièmement, d'autres systèmes fonctionnent par l'addition à l'aiguille ou au cône de prélèvement de deux électrodes flanquées, de part et d'autre de l'aiguille ou du cône, et assurant la conduction électrique lorsque ces électrodes entrent en contact avec le liquide biologique. Cette dernière variante présente le défaut majeur de contaminer les électrodes à chaque fois qu'elles entrent en contact avec un nouveau liquide biologique, nécessitant dans ce cas l'adjonction d'un système de lavage décontaminant.

Ce premier type de dispositif de l'art antérieur utilisant des propriétés capacitives nécessite toutefois des outils électriquement conducteurs.

Un deuxième type de dispositif de l'art antérieur permettant de détecter le contact de l'outil de prélèvement et d'une surface utilise des méthodes sonores. Ainsi, le brevet US-A-4, 846,003 décrit un système de détection qui utilise une caractéristique sonore émise par un haut-parleur et reçu par un microphone. La valeur mesurée est constituée par l'impédance acoustique.

Un troisième type de dispositif de l'art antérieur utilise des capteurs de pression (voir EP-A-0.341.438, US-A-5, 723, 795 et EP-A-0.571100). Plus précisément, une variation de la pression est détectée, dès que l'extrémité libre de l'embout arrive en contact avec la surface d'un liquide.

Enfin, un dernier dispositif de l'art antérieur concerne la détection d'une surface par ultrasons. Cette technique n'est toutefois pas adaptée aux petits éléments comme les colonies bactériennes.

Les documents US2006/043287A1 et DE102004030881A1 divulguent des procédés de positionnement de sondes dans les domaines de la microscopie électronique et de la fabrication de semi-conducteurs.

Toutefois, aucun des dispositifs de l'art antérieur précités ne permet d'obtenir un moyen « universel » du contrôle du déplacement d'un outil de prélèvement vers une surface de référence et/ou d'une surface de référence vers un outil prélèvement, pouvant être utilisé quel que soit le type d'outil de prélèvement employé et quelle que soit la nature ou la consistance de ladite surface de référence, tout en assurant un contrôle efficace du déplacement de l'outil de prélèvement vers la surface de référence et/ou de la une surface de référence vers l'outil prélèvement.

Ainsi la présente invention a pour objet un procédé selon la revendication

Par « piloter le déplacement d'un outil de prélèvement vers une surface de référence et/ou d'une surface de référence vers un outil de prélèvement », l'on entend, au sens de la présente invention, commander/diriger le déplacement de l'outil de prélèvement vers la surface de référence et/ou de la surface de référence vers l'outil de prélèvement. Le terme « piloter » doit être compris au sens large, à savoir comme englobant les notions de déclenchement et d'arrêt du déplacement ainsi que celles d'accélération et de ralentissement dudit déplacement.

En tout état de cause, le déplacement de l'outil de prélèvement vers la surface de référence et/ou de la surface de référence vers l'outil de prélèvement résulte en un « rapprochement » entre l'outil de prélèvement et la surface de référence.

Ce « rapprochement » peut être obtenu par :
- un déplacement de l'outil de prélèvement vers la surface de référence,
- un déplacement de la surface de référence vers l'outil de prélèvement, ou
- par un déplacement simultané ou successif de l'outil de prélèvement vers la surface de référence et de la surface de référence vers l'outil de prélèvement.

Selon un mode de réalisation préféré, le susdit « rapprochement » résulte d'un déplacement de l'outil de prélèvement vers la surface de référence.

Lorsque, sur l'image traitée obtenue à l'étape c) *supra,* la distance entre l'outil de prélèvement et son image projetée est égale à zéro, cela signifie que la position de contact est atteinte, à savoir que l'outil de prélèvement et la surface de référence sont en contact.

La surface de référence peut être substantiellement - voire totalement - plane, par exemple lorsqu'il s'agit d'un milieu liquide. Alternativement cette surface de référence peut comprendre des reliefs, tels que des zones surélevées (protubérances). Ces zones surélevées peuvent, par exemple, correspondre à des colonies bactériennes ayant poussé à la surface d'une gélose.

Préférablement, le procédé selon l'invention comprend les étapes complémentaires suivantes :
a') éclairer la surface de référence en l'absence de l'outil de prélèvement dans le champ,
b') acquérir une image de ladite surface de référence en l'absence de l'outil de prélèvement dans le champ, afin d'obtenir une image de référence;
et dans lequel l'étape de traitement d'image c) comprend la soustraction des éléments constituant l'image de référence à l'image de l'ensemble de l'outil de prélèvement et de son image projetée sur la surface de référence afin de conserver une image représentant essentiellement l'outil de prélèvement et son image projetée sur la surface de référence.

L'on entend par « soustraction des éléments constituant l'image de référence à l'image de l'outil de prélèvement et de son image projetée sur la surface de référence » tous les éléments de l'image de référence autres que l'image de l'outil de prélèvement et son image projetée sur la surface de référence.

L'éclairage de l'outil de prélèvement peut être maintenu pendant le déplacement de ce dernier (éclairage continu). Alternativement, l'éclairage de l'outil de prélèvement peut être ponctuel, à savoir ne se produire qu'après chaque déplacement de l'outil de prélèvement, de manière à projeter l'image de prélèvement sur la surface de référence pour chacune des différentes positions de l'outil de prélèvement.

De préférence, le procédé selon l'invention comprend l'étape complémentaire suivante :
e) lorsque ladite distance entre l'outil de prélèvement et son image projetée est égale à zéro, déplacer ou poursuivre le déplacement de l'outil de prélèvement et/ou de la surface de référence de la position de contact vers une position de prélèvement.

La position de prélèvement doit être comprise comme étant une position apte à effectuer le prélèvement de la matière biologique d'intérêt en évitant de prélever de possibles contaminants. Selon un mode de réalisation préféré, cette position de prélèvement se situe sensiblement sous la surface de référence.

Le déplacement ou la poursuite du déplacement de l'outil de prélèvement et/ou de la surface de référence de la position de contact vers une position de prélèvement est particulièrement souhaitable si l'outil de prélèvement doit être positionné sous la surface de référence, par exemple en cas de prélèvement de matière biologique en milieu liquide.

Selon un mode de réalisation alternatif, le procédé selon l'invention comprend l'étape complémentaire suivante :
e') Lorsque ladite distance entre l'outil de prélèvement et son image projetée est égale à zéro, stopper le déplacement de l'outil de prélèvement et/ou de la surface de référence.

Ce mode de réalisation alternatif peut présenter un intérêt notamment lorsque l'on prélève une matière biologique solide ou semi-solide (telle qu'une matière biologique bactérienne sur milieu gélosé) en faible quantité.

De préférence, à l'étape c) du procédé selon la présente invention, la distance entre l'outil de prélèvement et son image projetée sur la surface de référence est mesurée sur l'image traitée entre l'extrémité distale de l'outil de prélèvement et l'extrémité correspondante de son image projetée.

L'extrémité distale de l'outil de prélèvement peut notamment consister en l'extrémité d'un embout de prélèvement destiné à entrer en contact avec la matière biologique à prélever.

Selon un mode de réalisation particulier, le déplacement de l'outil de prélèvement vers la surface de référence, d'une première position vers une deuxième position, et/ou de la surface de référence vers l'outil de prélèvement, d'une première position vers une deuxième position, s'effectue en au moins deux étapes.

Selon un autre mode de réalisation particulier, le déplacement de l'outil de prélèvement et/ou de la surface de référence s'effectue à au moins deux vitesses différentes. Par exemple, un premier déplacement de l'outil de prélèvement et/ou de la surface de référence s'effectue à une première vitesse puis un deuxième déplacement s'effectue à une deuxième vitesse, ladite première vitesse étant supérieure à ladite deuxième vitesse.

De préférence, le déplacement de l'outil de prélèvement et/ou de la surface de référence est contrôlé par un moteur pas à pas. Cela autorise un « rapprochement » progressif de l'outil de prélèvement et de la surface de référence.

Selon un mode de réalisation particulièrement préféré, l'étape de traitement d'image c) comprend une binarisation de l'image, par exemple une binarisation à deux seuils, afin d'isoler, sur l'image traitée, l'image de l'outil de prélèvement et de son image projetée.

Selon un mode de réalisation préféré de la présente invention, l'image projetée de l'outil de prélèvement sur la surface de référence est un reflet. Ce reflet est obtenu par la réflexion de l'image de l'outil de prélèvement sur la surface de référence (effet miroir). Le reflet de l'outil de prélèvement peut notamment être obtenu sur des géloses claires et opaques, sous éclairages diffus.

Selon un autre mode de réalisation préféré, l'image projetée de l'outil de prélèvement sur la surface de référence est une ombre, à savoir l'ombre de l'outil de prélèvement sur ladite surface de référence. Afin de générer cette ombre projetée sur la surface de référence, l'outil de prélèvement est de préférence éclairé avec une source lumineuse pourvue d'un polariseur.

Préférablement, l'outil de prélèvement est éclairé avec une source lumineuse émettant un faisceau lumineux, ledit faisceau lumineux étant incliné d'un angle de Brewster par rapport à la surface de référence.

De préférence, l'outil de prélèvement est éclairé avec une source lumineuse de type LED, par exemple de couleur blanche.

La présente invention a également pour objet un dispositif selon la revendication 15.

Le susdit moyen de commande est adapté pour commander/piloter le déplacement de l'outil de prélèvement vers la surface de référence et/ou de la surface de référence vers l'outil de prélèvement. Le déplacement en tant que tel sera effectué à proprement parler par le susdit moyen de déplacement.

Préférablement, le moyen de commande est pourvu d'une mémoire pour stocker une distance entre l'outil de prélèvement et son image projetée égale à zéro, ledit moyen de commande étant adapté pour commander le déplacement dudit outil de prélèvement vers ladite surface de référence et/ou de ladite surface de référence vers ledit outil de prélèvement jusqu'à obtention, sur ladite image traitée, d'une distance égale à zéro entre l'outil de prélèvement et son image projetée.

Un autre objet de la présente invention concerne un appareil de prélèvement de matière biologique comprenant le dispositif selon l'invention.

L'invention apparaîtra plus clairement à la lecture de la description qui suit, faite en référence aux figures correspondantes, et représentant, à titre d'exemple non limitatif, un procédé et un dispositif selon la présente invention. Plus précisément :
- la figure 1 montre les différentes étapes d'un premier mode de réalisation d'un procédé pour piloter le déplacement d'un outil de prélèvement vers une surface de référence selon l'invention,
- la figure 2 représente un deuxième mode de réalisation d'un procédé permettant de piloter le déplacement d'un outil de prélèvement vers une surface de référence selon l'invention,
- la figure 3 montre, de façon schématique, les différents éléments d'un dispositif adapté pour piloter le déplacement d'un outil de prélèvement vers une surface de référence; l'outil de prélèvement étant positionné dans une première position par rapport à la surface de référence,
- la figure 4 représente, de façon schématique, le dispositif selon la figure 3, dans lequel l'outil de prélèvement est positionné dans une deuxième position (position de contact) par rapport à la surface de référence,
- la figure 5 montre, de façon schématique, l'image obtenue grâce à un moyen d'acquisition d'image tel qu'une caméra, après soustraction d'image et binarisation; l'outil de prélèvement étant positionné par rapport à la surface de référence tel que montré en figure 3,
- la figure 6 représente, toujours de façon schématique, l'image obtenue grâce à un moyen d'acquisition d'image tel qu'une caméra, après soustraction d'image et binarisation; l'outil de prélèvement étant positionné par rapport à la surface de référence comme montré en figure 4.

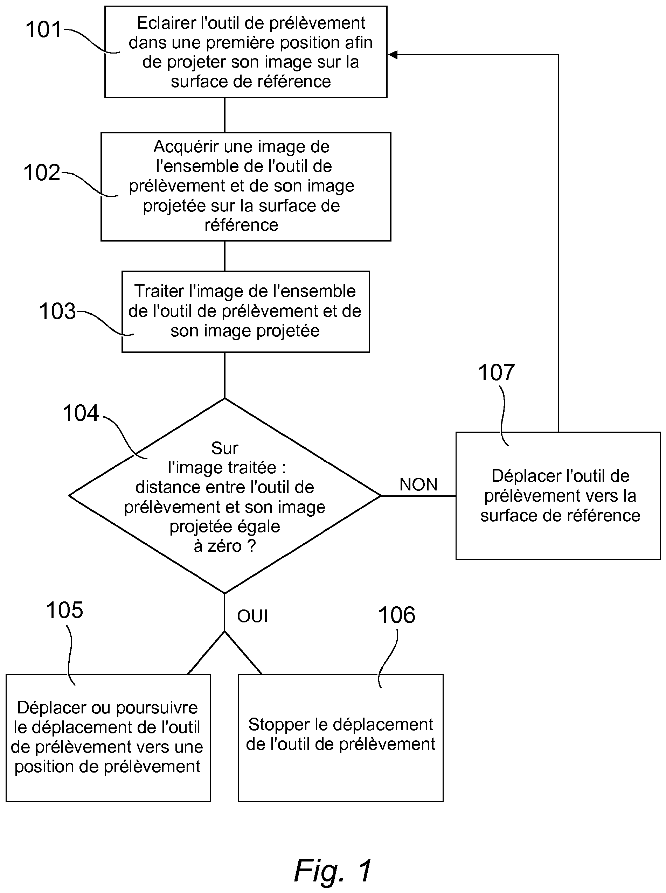

Les différentes étapes d'un premier mode de réalisation du procédé selon la présente invention sont représentées sur la figure 1. La première étape 101 consiste à éclairer l'outil de prélèvement dans une première position afin de rendre l'outil de prélèvement visible et projeter une image de l'outil de prélèvement sur la surface de référence, cette dernière étant par exemple une boîte de Pétri. L'image ainsi obtenue est appelée « image projetée ». Tel qu'indiqué précédemment, l'image projetée peut être une ombre obtenue sous éclairage collimaté (éclairage constitué par un faisceau de rayons lumineux parallèles) ou à un reflet obtenu sous éclairage diffus.

Une fois que l'outil de prélèvement est éclairé - et donc visible - pour créer cette image projetée sur la surface de référence, une image de l'ensemble de l'outil de prélèvement et de son image projetée sur la surface de référence est acquise à l'étape 102. Cette image est, par exemple, obtenue en utilisant un moyen d'acquisition d'image adapté, tel qu'une caméra.

Selon l'invention, l'image obtenue à l'étape 102 est ensuite traitée/analysée à l'étape 103, de préférence par une binarisation de l'image, par exemple une binarisation à deux seuils afin d'isoler, sur l'image traitée, l'image de l'outil de prélèvement et de son image projetée.

L'étape 104 consiste à vérifier si, sur l'image traitée à l'étape 103, la distance entre l'outil de prélèvement et son image projetée est égale à zéro (distance nulle ou non nulle).

Si cette distance est égale à zéro, la position de contact est atteinte. Dans ce cas de figure, en fonction des desiderata de l'utilisateur et des conditions de prélèvement (par exemple du type de milieu, milieu solide, milieu liquide, etc.) :
- l'outil de prélèvement est déplacé ou son déplacement est poursuivi vers une position de prélèvement à l'étape 105 ; ou
- le déplacement de l'outil de prélèvement est stoppé à l'étape 106.

En revanche, si, à l'étape 104, la distance entre l'outil de prélèvement et son image projetée n'est pas égale à zéro, cela signifie que la position de contact n'est pas atteinte, l'outil de prélèvement est déplacé vers la surface de référence à l'étape 107, puis les étapes 101, 102 103 et 104 sont répétées jusqu'à ce que, sur l'image traitée à l'image 103, la distance susvisée soit égale à zéro, ce qui signifie que l'outil de prélèvement et la surface de référence sont en contact (position de contact).

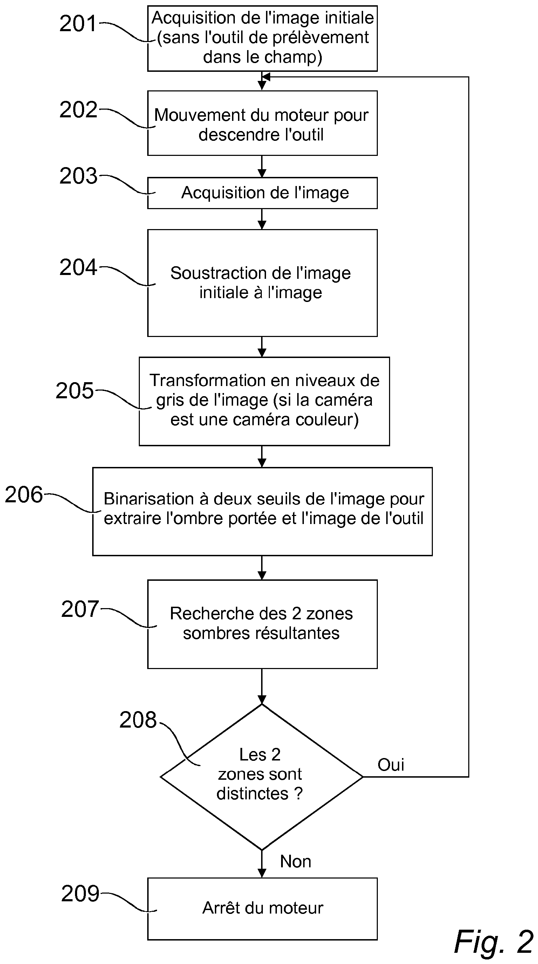

Un deuxième mode de réalisation du procédé selon l'invention est représenté en figure 2. Dans ce mode de réalisation, l'image de l'outil de prélèvement projetée sur la surface de référence est une ombre. Bien évidemment, ce deuxième mode de réalisation pourrait être adapté à une image projetée de type reflet.

Une acquisition d'image du champ, dans lequel l'outil de prélèvement est utilisé, est effectuée lors d'une première étape 201. Cette image de référence est acquise en l'absence de l'outil de prélèvement, à savoir sans que l'outil de prélèvement ne soit présent dans le champ. Ladite image peut être utilisée ultérieurement dans le présent procédé, lors de l'étape de soustraction d'image 204 (dans le cadre du processus de traitement d'image ; cf. ci-après).

Après acquisition de l'image initiale, l'outil de prélèvement est amené dans le champ du moyen d'acquisition d'images (par exemple une caméra).Plus précisément, un moteur (de préférence un moteur pas à pas) est utilisé pour effectuer la descente de l'outil à l'étape 202 et permettre son entrée dans le champ du moyen d'acquisition d'image. Lorsque l'outil de prélèvement est entré dans le champ de la caméra, il est éclairé. L'image de l'outil de prélèvement et de son ombre sur la surface de référence est acquise à l'étape 203.

A l'étape 204, l'image de référence obtenue à l'étape 201 est soustraite à l'image acquise à l'étape 203, afin de visualiser uniquement sur l'image résultante l'outil de prélèvement et son ombre portée.

Après cette première étape de traitement d'image, l'image ainsi obtenue (image résultante) est transformée en niveaux de gris dans l'éventualité où la caméra est une caméra couleur. Cette transformation est décrite à l'étape 205.

Cette opération de traitement d'image par « soustraction » permet d'obtenir une image résultante ne contenant que l'image de l'outil de prélèvement et de son image projetée sur la surface de référence. Afin d'obtenir ce résultat, cette opération de soustraction consiste, selon un mode de réalisation préféré, à comparer chaque niveau de gris de chaque pixel de l'image acquise au niveau de gris du pixel correspondant dans l'image de référence. Les niveaux de gris sont au nombre de 255. Si les niveaux de gris de ces pixels sont identiques, le niveau de gris du pixel dans l'image résultante sera égal à 255 (niveau correspondant à la couleur blanche); si les niveaux de gris de ces pixels sont différents, le niveau de gris du pixel dans l'image résultante sera égal à zéro (niveau correspondant à la couleur noire).

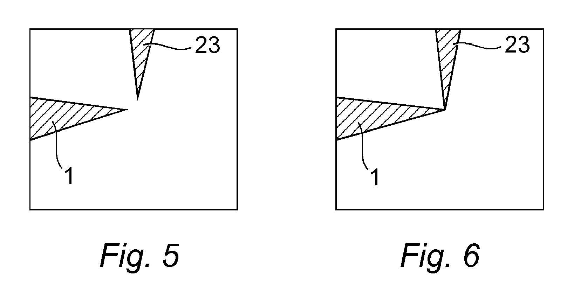

L'étape suivante 206 du processus de traitement d'image consiste en la binarisation à deux seuils, haut et bas, de l'image afin d'extraire l'image de l'outil de prélèvement et de son ombre portée. L'image ainsi binarisée permet de distinguer clairement l'image de l'outil de prélèvement et de son ombre portée. Sur ladite image binarisée, à la fois l'outil de prélèvement et son ombre portée apparaissent comme deux zones sombres, tel que représenté dans les figures 5 et 6.

L'étape suivante 207 consiste en une étape d'analyse d'image où l'on recherche les deux zones sombres, représentant respectivement l'outil de prélèvement et son ombre portée, tel qu'indiqué précédemment. Lorsque les deux zones sombres sont identifiées, l'on contrôle si ces deux zones sont distinctes, à savoir s'il existe une distance entre ces deux zones.

Dans le cas où subsiste une certaine distance entre les deux zones susvisées, les étapes 202 à 208 sont répétées jusqu'à ce que les deux zones sombres soient au contact l'une de l'autre (il n'existe plus d'espace entre ces deux zones). Dans le cas contraire, si les deux zones sombres sont au contact l'une de l'autre, cela indique que l'outil de prélèvement est entré en contact avec la surface de référence. En d'autres termes, l'outil de prélèvement est arrivé à sa position de contact. Le déplacement de l'outil de prélèvement en direction de la surface de référence est stoppé à l'étape 209, par exemple par arrêt du moteur entraînant l'outil de prélèvement. Comme mentionné précédemment, un déplacement de l'outil de prélèvement de cette position de contact vers une position de prélèvement (définie ci-dessus) peut être effectué selon les souhaits de l'utilisateur. Selon une alternative, le moteur n'est pas stoppé à l'étape 209 et le déplacement de l'outil de prélèvement est poursuivi jusqu'à la position de prélèvement soit atteinte.

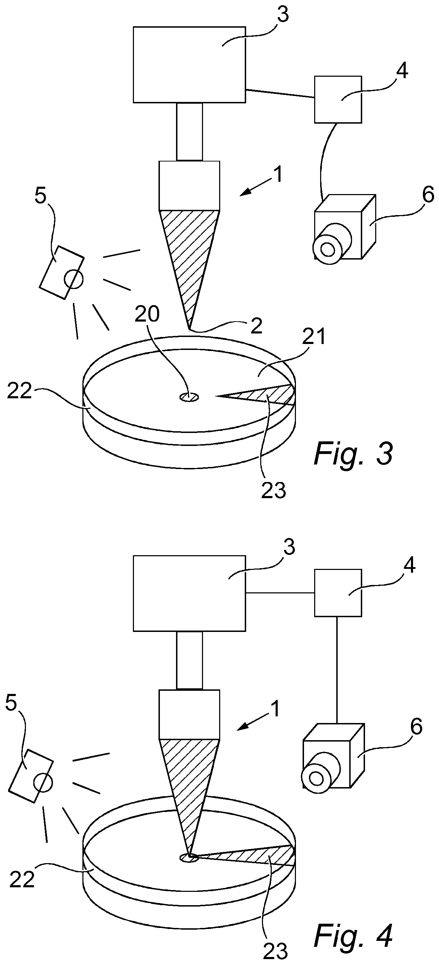

La figure 3 représente, de façon schématique, un dispositif permettant de piloter le déplacement d'un outil de prélèvement vers une surface de référence selon la présente invention.

Un outil de prélèvement 1, pourvu d'un embout 2 destiné à entrer en contact avec une surface de référence est représenté sur la figure 3. L'outil de prélèvement 1 peut être déplacé d'une première position vers une deuxième position en utilisant un moteur 3, de préférence un moteur pas à pas. Le moteur 3 est connecté à un dispositif de commande 4 permettant de commander/piloter le mouvement de l'outil de prélèvement 1 vers la position de contact ainsi que, le cas échéant, vers la position de prélèvement.

Tel que représenté en figure 3, la position de contact de l'embout 2 et de l'outil de prélèvement 1 correspond à un contact entre ledit embout 2 et une colonie bactérienne 20 présente sur un milieu de culture gélosé 21 contenu dans une boîte de Pétri, dont le fond est référencé 22. Au sens de la présente invention, la surface de référence comprend, dans cette configuration, le milieu de culture gélosé 21 et la colonie bactérienne 20.

Il est important de piloter minutieusement le mouvement de l'outil de prélèvement 1 vers la colonie bactérienne 20 afin de ne prélever que des bactéries appartenant à cette colonie bactérienne 20, ceci afin d'éviter les phénomènes de contamination mentionnés précédemment. Afin d'optimiser l'analyse de la colonie bactérienne 20, le prélèvement de bactéries appartenant à cette colonie 20 sera effectué, de préférence, dans la position de contact, afin d'éviter tout prélèvement de le milieu de culture gélosé 21. Dans ce cas de figure particulier, les positions de contact et de prélèvement sont confondues. De manière optimale, ce prélèvement contiendra uniquement des bactéries appartenant à la colonie 20 et aucun autre matériau.

Selon l'invention, le pilotage du déplacement de l'outil de prélèvement en direction de la surface de référence est effectué en utilisant une source lumineuse 5, laquelle crée une ombre 23, encore appelée « ombre portée » sur la surface de référence.

Une fois l'ombre portée 23 créée, l'on utilise une caméra 6, ou tout autre moyen d'acquisition d'image adapté, afin d'obtenir une image de l'outil de prélèvement 1 et de son ombre portée 23 sur la surface de référence.

L'image ainsi obtenue par la caméra 6 est ensuite traitée lors d'une ou plusieurs étapes du traitement d'image (cf. figure 2, étapes 204 à 206). L'image ainsi traitée est représentée en figure 5. L'on aperçoit, sur ladite figure 5, les deux zones sombres obtenues à l'issue de l'étape 206 (figure 2). Ces deux zones sombres représentent respectivement l'outil de prélèvement 1 et son ombre portée 23 sur la surface de référence.

Une distance entre les deux zones sombres représentant respectivement l'outil de prélèvement 1 et son ombre portée 23 est visible sur la figure 5. Cette distance signifie que l'embout 2 de l'outil de prélèvement 1 et la colonie bactérienne 20 présente sur la gélose 21 ne sont pas en contact.

L'image traitée telle que représentée sur la figure 5 est analysée (cf. étapes 207 et 208 de la figure 2), le résultat de cette analyse pouvant donner lieu à des ordres de commande (pilotage) émis d'un moyen de commande 4 à destination du moteur 3. Le moyen de commande 4 peut, par exemple, piloter la descente de l'outil de prélèvement 1 en direction de la surface de référence jusqu'à ce que l'embout 2 de l'outil de prélèvement 1 soit en contact avec la colonie bactérienne 20. Ce contact signifie que l'outil de prélèvement 1 se trouve en position de contact. Comme mentionné précédemment, cette position de contact peut également être la position de prélèvement, dans laquelle un prélèvement sera effectué. Alternativement, la position de prélèvement peut être distincte de cette position de contact, en fonction de la nature de la matière biologique à prélever ainsi que du type de milieu de prélèvement (solide, liquide, etc.).

Selon un mode de réalisation préférentiel, l'outil de prélèvement descend par étapes en direction de la surface de référence. Après chaque étape, on obtient une nouvelle image de l'outil de prélèvement 1 et de son ombre portée 23. Le moyen de commande 4 continue d'ordonner au moteur 3 de poursuivre la descente de l'outil de prélèvement 1 jusqu'au moment où l'on constate, sur l'image traitée, que les deux zones sombres représentant respectivement l'outil de prélèvement 1 et son ombre portée 23 sur la surface de référence sont au contact, à savoir que la distance entre les deux zones sombres est nulle. Cette situation est représentée en figures 4 et 6.

Revenant à la figure 4, il apparaît clairement que le moteur 3 a entraîné la descente de l'outil de prélèvement 1 jusqu'à ce que l'embout 2 soit en contact avec la colonie bactérienne 20 présente sur le milieu de culture gélosé 21. Comme mentionné précédemment, l'outil de prélèvement est désormais en position de contact et le prélèvement de colonies bactériennes peut donc être effectué dans cette position de contact ou dans une position de prélèvement distincte de cette dernière, en fonction des souhaits de l'opérateur. L'image de cette position dite « de contact », obtenue par la caméra 6, à l'étape 203, et traitée lors des étapes de traitement d'images 204 à 206, est représentée en figure 6.

Sur l'image traitée représentée en figure 6, l'on distingue clairement une première zone sombre, représentant l'outil de prélèvement 1 et une deuxième zone sombre représentant son ombre sur la surface de référence (ombre portée); les deux zones sombres faisant contact l'une avec l'autre. Cela signifie que l'outil de prélèvement 1 a atteint sa position de contact, à savoir qu'il est entré en contact avec la colonie bactérienne 20 présente sur le milieu de culture gélosé 21, et que sa descente peut donc être stoppée ou poursuivie, dans un même déplacement ou un déplacement subséquent, jusqu'à ce que la position de prélèvement soit atteinte.

En fonction de l'utilisation souhaitée de l'outil de prélèvement, le fait que, sur l'image traitée, les deux zones sombres entrent en contact l'une avec l'autre peut être une indication selon laquelle le moyen de commande 4 doit stopper le déplacement de l'outil de prélèvement 1. Toutefois, par exemple dans le cas d'un prélèvement en milieu liquide, il peut être souhaitable que l'outil de prélèvement poursuive légèrement sa descente sous le niveau de référence (à savoir sous la surface du liquide à prélever) jusqu'à une position de prélèvement, afin, par exemple, d'éviter tout prélèvement de fluides environnementaux, tel que tout prélèvement d'air ambiant.

Tout comme la figure 2, les figures 3, 4, 5 et 6 concernent un mode de réalisation dans lequel l'image de l'outil de prélèvement projetée sur la surface de référence est une ombre. Bien évidemment, l'homme du métier comprendra que ces figures pourraient aisément être adaptées au mode de réalisation dans lequel l'image projetée est un reflet.

En faisant référence au procédé et au dispositif décrit ci-dessus, il convient de noter que, selon la présente invention, une grande variété d'outils de prélèvement peut être utilisée. Un outil de prélèvement adéquat peut consister en un outil de prélèvement « passif », par exemple un outil pourvu en son extrémité d'un élément en coton destiné à entrer en contact avec une colonie bactérienne.

De façon alternative, l'on peut utiliser des outils de prélèvement dits « actifs », par exemple des pipettes utilisant le principe d'aspiration/refoulement.

Selon la présente invention, un moyen d'éclairage 5, tel qu'une lampe, est utilisé pour éclairer l'outil de prélèvement 1 et créer son ombre portée 23 sur une surface de référence. Cet éclairage peut être assuré par une ou plusieurs LED, par exemple une ou plusieurs LED de couleur blanche. La couleur des LED peut toutefois varier en fonction des utilisations souhaitées. Ce type d'éclairage peut être utilisé pour assurer un contraste suffisant entre l'outil de prélèvement 1 et son ombre portée 23.

En outre, l'orientation du moyen d'éclairage 5 par rapport à l'outil de prélèvement 1 a un effet sur les reflets parasites éventuels de la lumière présente sur la surface de référence.

Selon un mode de réalisation particulièrement préféré, et ce afin de favoriser l'acquisition d'images et de supprimer les artéfacts dus au reflet parasite de la source lumineuse sur la surface de référence, il est possible de placer le moyen d'éclairage 5 de telle sorte que le faisceau d'éclairage soit incliné d'un angle de Brewster par rapport à la surface de référence. En outre, il est également recommandé d'utiliser un polariseur devant la source lumineuse afin de réduire les reflets sur la surface de référence et, le cas échéant, de minimiser la création d'une deuxième ombre portée au fond d'une boîte de Pétri 22, dans l'éventualité où l'on utilise une gélose translucide.

L'apparition de cette deuxième ombre au fond de la boîte de Pétri 22 peut également être éliminée lors de l'étape de traitement d'images par binarisation car moins intense et décalée par rapport à l'ombre portée 23 sur la surface de référence.

En outre, il convient de noter que l'étape 206, représentée en figure 2, concerne une binarisation à deux seuils. De manière intéressante, une binarisation à un seul seuil est suffisante si l'embout 2 de l'outil de prélèvement 1 est de couleur sombre, par exemple de couleur noire.

Le dispositif pour piloter le déplacement d'un outil de prélèvement vers une surface de référence et/ou d'une surface de référence vers l'outil de prélèvement selon l'invention est, de manière avantageuse, inclus dans un appareil de prélèvement de matière biologique, de préférence automatisé. De manière préférée, cet appareil de prélèvement sera en fait un automate d'identification et/ou d'antibiogramme, tel qu'un appareil de type VITEK® VITEK® 2, ou VITEK® 2 Compact, ou équivalent.

## Revendications

1. Procédé pour effectuer un prélèvement de matière biologique sur une surface de référence, telle que la surface d'un milieu gélosé, à l'aide d'un outil de prélèvement, ledit procédé permettant de piloter le déplacement de l'outil de prélèvement vers la surface de référence, d'une première position vers une deuxième position, et/ou de la surface de référence vers l'outil prélèvement, d'une première position vers une deuxième position, ladite deuxième position étant une position de contact dans laquelle l'outil de prélèvement et la surface de référence sont en contact, ledit procédé comprenant les étapes suivantes :
a) éclairer l'outil de prélèvement dans ladite première position afin de rendre l'outil de prélèvement visible et de projeter une image de l'outil de prélèvement sur la surface de référence,
b) acquérir une image de l'ensemble de l'outil de prélèvement et de son image projetée sur la surface de référence,
c) traiter l'image obtenue à l'étape b) afin de déterminer si, sur l'image traitée, la distance entre l'outil de prélèvement et son image projetée est égale à zéro,
d) si la distance entre l'outil de prélèvement et son image projetée n'est pas égale à zéro, déplacer l'outil de prélèvement vers la surface de référence et/ou la surface de référence vers l'outil de prélèvement et répéter les étapes a), b), c) et d) jusqu'à ce que ladite distance soit égale à zéro.

2. Procédé selon la revendication 1, dans lequel ledit procédé comprend les étapes suivantes :
a') éclairer la surface de référence en l'absence de l'outil de prélèvement dans le champ,
b') acquérir une image de ladite surface de référence en l'absence de l'outil de prélèvement dans le champ, afin d'obtenir une image de référence ;
et dans lequel l'étape de traitement d'image c) comprend la soustraction des éléments constituant l'image de référence à l'image de l'ensemble de l'outil de prélèvement et de son image projetée sur la surface de référence afin de conserver une image représentant essentiellement l'outil de prélèvement et son image projetée sur la surface de référence.

3. Procédé selon la revendication 1 ou 2, ledit procédé comprenant les étapes suivantes :
e) lorsque ladite distance entre l'outil de prélèvement et son image projetée est égale à zéro, déplacer ou poursuivre le déplacement de l'outil de prélèvement et/ou de la surface de référence de la position de contact vers une position de prélèvement.

4. Procédé selon la revendication 1 ou 2, ledit procédé comprenant l'étape suivante :
e') Lorsque ladite distance entre l'outil de prélèvement et son image projetée est égale à zéro, stopper le déplacement de l'outil de prélèvement et/ou de la surface de référence.

5. Procédé selon l'une des revendications 1 à 4, dans lequel, à l'étape c), la distance entre l'outil de prélèvement et son image projetée sur la surface de référence est mesurée sur l'image traitée entre l'extrémité distale de l'outil de prélèvement et l'extrémité correspondante de son image projetée.

6. Procédé selon l'une des revendications 1 à 5, dans lequel le déplacement de l'outil de prélèvement vers la surface de référence, d'une première position vers une deuxième position, et/ou de la surface de référence vers l'outil de prélèvement, d'une première position vers une deuxième position, s'effectue en au moins deux étapes.

7. Procédé selon l'une des revendications 1 à 6, dans lequel le déplacement de l'outil de prélèvement et/ou de la surface de référence s'effectue à au moins deux vitesses différentes.

8. Procédé selon l'une des revendications 1 à 7, dans lequel le déplacement de l'outil de prélèvement et/ou de la surface de référence est contrôlé par un moteur pas à pas.

9. Procédé selon l'une des revendications 1 à 8, dans lequel l'étape de traitement d'image c) comprend une binarisation de l'image, par exemple une binarisation à deux seuils, afin d'isoler, sur l'image traitée, l'image de l'outil de prélèvement et de son image projetée.

10. Procédé selon l'une des revendications 1 à 9, dans lequel l'image projetée de l'outil de prélèvement sur la surface de référence est un reflet.

11. Procédé selon l'une des revendications 1 à 9, dans lequel l'image projetée de l'outil de prélèvement sur la surface de référence est une ombre.

12. Procédé selon la revendication 11, dans lequel l'outil de prélèvement est éclairé avec une source lumineuse pourvue d'un polariseur.

13. Procédé selon la revendication 11 ou 12, dans lequel l'outil de prélèvement est éclairé avec une source lumineuse émettant un faisceau lumineux, ledit faisceau lumineux étant incliné d'un angle de Brewster par rapport à la surface de référence.

14. Procédé selon l'une des revendications 11 à 13, dans lequel l'outil de prélèvement est éclairé avec une source lumineuse de type LED, par exemple de couleur blanche.

15. Dispositif pour la mise en œuvre du procédé selon l'une des revendications 1 à 14, ledit dispositif comprenant :
- un outil de prélèvement adapté pour effectuer un prélèvement de matière biologique sur une surface de référence, telle que la surface d'un milieu gélosé,
- un moyen d'éclairage pour rendre l'outil de prélèvement visible et projeter une image de l'outil de prélèvement sur la surface de référence,
- un moyen d'acquisition d'image tel qu'une caméra, permettant d'acquérir une image de l'ensemble de l'outil de prélèvement et de son image projetée sur la surface de référence,
- un moyen de traitement d'image permettant de traiter l'image et déterminer si, sur l'image traitée, la distance entre l'outil de prélèvement et son image projetée est égale à zéro,
- un moyen de déplacement, tel qu'un moteur, permettant le déplacement de l'outil de prélèvement vers la surface de référence et/ou de la surface de référence vers l'outil de prélèvement, et
- un moyen de commande, adapté pour commander le déplacement de l'outil de prélèvement vers la surface de référence et/ou de la surface de référence vers l'outil de prélèvement lorsque, sur ladite image traitée, la distance entre l'outil de prélèvement et son image projetée n'est pas égale à zéro.

16. Dispositif selon la revendication 15, dans lequel le moyen de commande est pourvu d'une mémoire pour stocker une distance entre l'outil de prélèvement et son image projetée égale à zéro, et dans lequel ledit moyen de commande est adapté pour commander le déplacement dudit outil de prélèvement vers ladite surface de référence et/ou de ladite surface de référence vers ledit outil de prélèvement jusqu'à obtention, sur ladite image traitée, d'une distance égale à zéro entre l'outil de prélèvement et son image projetée.

17. Appareil de prélèvement de matière biologique comprenant le dispositif selon la revendication 15 ou 16.

## Patentansprüche

1. Verfahren zur Durchführung einer Entnahme biologischen Materials auf einer Referenzoberfläche, wie der Oberfläche eines Agarmediums, mithilfe eines Entnahmewerkzeugs, wobei es das Verfahren gestattet, die Bewegung des Entnahmewerkzeugs zur Referenzoberfläche von einer ersten Position zu einer zweiten Position und/oder der Referenzoberfläche zum Entnahmewerkzeug von einer ersten Position zu einer zweiten Position zu steuern, wobei die zweite Position eine Kontaktposition ist, in welcher das Entnahmewerkzeug und die Referenzoberfläche in Kontakt stehen, das Verfahren umfassend die folgenden Schritte:
a) Beleuchten des Entnahmewerkzeugs in der ersten Position, um das Entnahmewerkzeug sichtbar zu machen und ein Bild des Entnahmewerkzeugs auf die Referenzoberfläche zu projizieren,
b) Erfassen eines Bilds der Gesamtheit des Entnahmewerkzeugs und dessen Bilds, das auf die Referenzoberfläche projiziert wird,
c) Verarbeiten des Bilds, das bei Schritt b) erhalten wird, um zu bestimmen, ob auf dem verarbeiteten Bild der Abstand zwischen dem Entnahmewerkzeug und dessen projizierten Bild gleich null ist,
d) wenn der Abstand zwischen dem Entnahmewerkzeug und dessen projizierten Bild nicht gleich null ist, Bewegen des Entnahmewerkzeugs zur Referenzoberfläche und/oder der Referenzoberfläche zum Entnahmewerkzeug und Wiederholen der Schritte a), b), c) und d), bis der Abstand gleich null ist.

2. Verfahren nach Anspruch 1, wobei das Verfahren die folgenden Schritte umfasst:
a') Beleuchten der Referenzoberfläche, wobei das Entnahmewerkzeug im Feld fehlt,
b') Erfassen eines Bilds der Referenzoberfläche, wobei das Entnahmewerkzeug im Feld fehlt, um ein Referenzbild zu erhalten;
und wobei der Schritt des Verarbeitens des Bilds c) die Subtraktion der Elemente, die das Referenzbild bilden, vom Bild der Gesamtheit des Entnahmewerkzeugs und dessen Bilds, das auf die Referenzoberfläche projiziert wird, umfasst, um ein Bild zu behalten, das im Wesentlichen das Entnahmewerkzeug und dessen Bild, das auf die Referenzoberfläche projiziert wird, darstellt.

3. Verfahren nach Anspruch 1 oder 2, wobei das Verfahren die folgenden Schritte umfasst:
e) wenn der Abstand zwischen dem Entnahmewerkzeug und dessen projizierten Bild gleich null ist, Bewegen oder Fortsetzen der Bewegung des Entnahmewerkzeugs und/oder der Referenzoberfläche von der Kontaktposition zu einer Entnahmeposition.

4. Verfahren nach Anspruch 1 oder 2, wobei das Verfahren den folgenden Schritt umfasst:
e') Wenn der Abstand zwischen dem Entnahmewerkzeug und dessen projizierten Bild gleich null ist, Anhalten der Bewegung des Entnahmewerkzeugs und/oder der Referenzoberfläche.

5. Verfahren nach einem der Ansprüche 1 bis 4, wobei bei Schritt c) der Abstand zwischen dem Entnahmewerkzeug und dessen Bild, das auf die Referenzoberfläche projiziert wird, auf dem verarbeiteten Bild zwischen dem distalen Ende des Entnahmewerkzeugs und dem entsprechenden Ende dessen projizierten Bilds gemessen wird.

6. Verfahren nach einem der Ansprüche 1 bis 5, wobei die Bewegung des Entnahmewerkzeugs zur Referenzoberfläche von einer ersten Position zu einer zweiten Position und/oder der Referenzoberfläche zum Entnahmewerkzeug von einer ersten Position zu einer zweiten Position in wenigstens zwei Schritten erfolgt.

7. Verfahren nach einem der Ansprüche 1 bis 6, wobei die Bewegung des Entnahmewerkzeugs und/oder der Referenzoberfläche mit wenigstens zwei verschiedenen Geschwindigkeiten erfolgt.

8. Verfahren nach einem der Ansprüche 1 bis 7, wobei die Bewegung des Entnahmewerkzeugs und/oder der Referenzoberfläche von einem Motor Schritt für Schritt kontrolliert wird.

9. Verfahren nach einem der Ansprüche 1 bis 8, wobei der Schritt des Verarbeitens des Bilds c) eine Binarisierung des Bilds umfasst, zum Beispiel eine zweischwellige Binarisierung, um auf dem verarbeiteten Bild das Bild des Entnahmewerkzeugs und dessen projizierten Bilds zu isolieren.

10. Verfahren nach einem der Ansprüche 1 bis 9, wobei das projizierte Bild des Entnahmewerkzeugs auf der Referenzoberfläche eine Spiegelung ist.

11. Verfahren nach einem der Ansprüche 1 bis 9, wobei das projizierte Bild des Entnahmewerkzeugs auf der Referenzoberfläche ein Schatten ist.

12. Verfahren nach Anspruch 11, wobei das Entnahmewerkzeug mit einer Lichtquelle beleuchtet wird, die mit einem Polarisator versehen ist.

13. Verfahren nach Anspruch 11 oder 12, wobei das Entnahmewerkzeug mit einer Lichtquelle beleuchtet wird, die ein Lichtbündel abgibt, wobei das Lichtbündel um einen Brewster-Winkel bezogen auf die Referenzoberfläche geneigt ist.

14. Verfahren nach einem der Ansprüche 11 bis 13, wobei das Entnahmewerkzeug mit einer Lichtquelle vom LED-Typ beispielsweise weißen Lichts beleuchtet wird.

15. Vorrichtung zur Durchführung des Verfahrens nach einem der Ansprüche 1 bis 14, die Vorrichtung umfassend:
- ein Entnahmewerkzeug, das geeignet ist, eine Entnahme biologischen Materials auf einer Referenzoberfläche durchzuführen, wie der Oberfläche eines Agarmediums,
- ein Beleuchtungsmittel zum Sichtbarmachen des Entnahmewerkzeugs und Projizieren eines Bilds des Entnahmewerkzeugs auf die Referenzoberfläche,
- ein Bilderfassungsmittel wie eine Kamera, das es gestattet, ein Bild der Gesamtheit des Entnahmewerkzeugs und dessen Bilds, das auf die Referenzoberfläche projiziert wird, zu erfassen,
- ein Bildverarbeitungsmittel, das es gestattet, das Bild zu verarbeiten und zu bestimmen, ob auf dem verarbeiteten Bild der Abstand zwischen dem Entnahmewerkzeug und dessen projizierten Bild gleich null ist,
- ein Bewegungsmittel, wie ein Motor, der die Bewegung des Entnahmewerkzeugs zur Referenzoberfläche und/oder der Referenzoberfläche zum Entnahmewerkzeug gestattet, und
- ein Steuermittel, das geeignet ist, die Bewegung des Entnahmewerkzeugs zur Referenzoberfläche und/oder der Referenzoberfläche zum Entnahmewerkzeug zu steuern, wenn auf dem verarbeiteten Bild der Abstand zwischen dem Entnahmewerkzeug und dessen projizierten Bild nicht gleich null ist.

16. Vorrichtung nach Anspruch 15, wobei das Steuermittel mit einem Speicher versehen ist, um einen Abstand zwischen dem Entnahmewerkzeug und dessen projizierten Bild, der gleich null ist, zu speichern, und wobei das Steuermittel geeignet ist, die Bewegung des Entnahmewerkzeugs zur Referenzoberfläche und/oder der Referenzoberfläche zum Entnahmewerkzeug bis zum Erhalt, auf dem verarbeiteten Bild, eines Abstands gleich null zwischen dem Entnahmewerkzeug und dessen projizierten Bild zu steuern.

17. Gerät zur Entnahme biologischen Materials umfassend die Vorrichtung nach Anspruch 15 oder 16.

## Claims

1. Method for taking a sample of biological material from a reference surface, such as the surface of an agar medium, using a sampling tool, said method allowing the movement of the sampling tool toward the reference surface, from a first position to a second position, and/or from the reference surface toward the sampling tool, from a first position to a second position, said second position being a contact position in which the sampling tool and the reference surface make contact, to be controlled, said method comprising the following steps:
a) illuminating the sampling tool in said first position in order to make the sampling tool visible and to project an image of the sampling tool onto the reference surface,
b) acquiring an image of all of the sampling tool and of its image projected onto the reference surface,
c) processing the image obtained in step b) in order to determine whether, in the processed image, the distance between the sampling tool and its projected image is equal to zero,
d) if the distance between the sampling tool and its projected image is not equal to zero, moving the sampling tool toward the reference surface and/or the reference surface toward the sampling tool and repeating steps a), b), c) and d) until said distance is equal to zero.

2. Method according to Claim 1, wherein said method comprises the following steps:
a') illuminating the reference surface in the absence of the sampling tool from the field,
b') acquiring an image of said reference surface in the absence of the sampling tool from the field, in order to obtain a reference image;
and wherein the image-processing step, step c), comprises subtracting the elements making up the reference image from the image of all of the sampling tool and of its image projected onto the reference surface in order to preserve an image representing essentially the sampling tool and its image projected onto the reference surface.

3. Method according to Claim 1 or 2, said method comprising the following steps:
e) when said distance between the sampling tool and its projected image is equal to zero, moving or continuing the movement of the sampling tool and/or of the reference surface from the contact position to a sampling position.

4. Method according to Claim 1 or 2, said method comprising the following step:
e') when said distance between the sampling tool and its projected image is equal to 0, stopping the movement of the sampling tool and/or of the reference surface.

5. Method according to one of Claims 1 to 4, wherein, in step c), the distance between the sampling tool and its image projected onto the reference surface is measured in the processed image between the far end of the sampling tool and the corresponding end of its projected image.

6. Method according to one of Claims 1 to 5, wherein the movement of the sampling tool toward the reference surface, from a first position to a second position, and/or of the reference surface toward the sampling tool, from a first position to a second position, is carried out in at least two steps.

7. Method according to one of Claims 1 to 6, wherein the movement of the sampling tool and/or of the reference surface is carried out at at least two different speeds.

8. Method according to one of Claims 1 to 7, wherein the movement of the sampling tool and/or of the reference surface is controlled by a stepper motor.

9. Method according to one of Claims 1 to 8, wherein the image-processing step, step c), comprises binarizing the image, for example via double-threshold binarization, in order to isolate, in the processed image, the image of the sampling tool and of its projected image.

10. Method according to one of Claims 1 to 9, wherein the projected image of the sampling tool on the reference surface is a reflection.

11. Method according to one of Claims 1 to 9, wherein the projected image of the sampling tool on the reference surface is a shadow.

12. Method according to Claim 11, wherein the sampling tool is illuminated with a light source provided with a polarizer.

13. Method according to Claim 11 or 12, wherein the sampling tool is illuminated with a light source emitting a light beam, said light beam being inclined by a Brewster angle with respect to the reference surface.

14. Method according to one of Claims 11 to 13, wherein the sampling tool is illuminated with a light source of LED type, of white colour for example.

15. Device for implementing the method according to one of Claims 1 to 14, said device comprising:
- a sampling tool suitable for taking a sample of biological material from a reference surface, such as the surface of an agar medium,
- an illuminating means for making the sampling tool visible and projecting an image of the sampling tool onto the reference surface,
- an image-acquiring means such as a camera, allowing an image of all of the sampling tool and of its image projected onto the reference surface to be acquired,
- an image-processing means allowing the image to be processed and allowing it to be determined whether, in the processed image, the distance between the sampling tool and its projected image is equal to zero,
- a moving means, such as a motor, allowing the sampling tool to be moved toward the reference surface and/or the reference surface to be moved toward the sampling tool, and
- a control means, suitable for controlling the movement of the sampling tool toward the reference surface and/or of the reference surface toward the sampling tool when, in said processed image, the distance between the sampling tool and its projected image is not equal to zero.

16. Device according to Claim 15, wherein the control means is provided with a memory for storing a distance between the sampling tool and its projected image equal to zero, and wherein said control means is suitable for controlling the movement of said sampling tool toward said reference surface and/or of said reference surface toward said sampling tool until, in said processed image, a distance equal to zero between the sampling tool and its projected image is obtained.

17. Apparatus for sampling biological material comprising the device according to Claim 15 or 16.
